# FASCICULE DE BREVET EUROPEEN

(11) **EP 1 251 842 B1**
(45) Date de publication et mention de la délivrance du brevet: **27.08.2003**
(21) Numéro de dépôt: 01903990.8
(22) Date de dépôt: 29.01.2001
(51) Int. Cl.: A61K 31/165, A61P 25/26, A61P 21/00

(54) **UTILISATION DU MODAFINIL POUR L'OBTENTION D'UN MEDICAMENT DESTINE A CORRIGER LES TROUBLES DE LA VIGILANCE ASSOCIES AUX MYOPATHIES**
VERWENDUNG VON MODAFINIL ZUR HERSTELLUNG EINES ARZNEIMITTELS ZUR BEHANDLUNG VON VIGILANZSTÖRUNGEN IM ZUSAMMENHANG MIT MYOPATHIEN
USE OF MODAFINIL FOR THE MANUFACTURE OF A MEDICAMENT FOR CORRECTING VIGILANCE DISORDERS RELATED TO MYOPATHIES

(30) Priorité: 31.01.2000 FR 0001211
(43) Date de publication de la demande: 30.10.2002
(73) Titulaire: CEPHALON FRANCE, 94700 Maisons-Alfort (FR)
(72) Inventeur: BATTAGLIA, Fabrice, F-67100 Strasbourg (FR); KRIEGER, Jean, F-67000 Strasbourg (FR); PETIAU, Christophe, F-67000 Strasbourg (FR); VIAL, Christophe, F-69006 Lyon (FR); BASTUJI, Hélène, F-69007 Lyon (FR); LUBIN, Serge, F-94800 Villejuif (FR)
(74) Mandataire: Bernasconi, Jean
(86) Numéro de dépôt international: FR0100271
(87) Numéro de publication internationale: WO01054648

(56) Documents cités:
- EP-A- 0 233 106
- WO-A-01/12170
- FR-A- 2 385 693
- FR-A- 2 771 004
- US-A- 5 618 845
- MARCHAND: "Therapeutic results of modafinil in 32 patients out of 112 hypersomniacs examined at a sleep unit" NEUROPHYSIOL. CLIN., vol. 21, no. 3, 1991, page 227 XP000972147
- PETIAU: "Somnolence diurne pathologique" REVUE DE MEDECINE INTERNE, vol. 18, no. 3, 1997, pages 210-218, XP000972143
- BASTUJI: "Successful treatment of idiopathic hypersomnia and narcolepsy with modafinil" PROGRESS IN NEURO-PSYCHOPHARMACOLOGY AND BIOLOGICAL PSYCHIATRY, vol. 12, no. 5, 1988, pages 695-700, XP000972003 cité dans la demande
- ROCHEGUDE: "Le modafinil : Modiodal(R)" LYON PHARMACEUTIQUE, vol. 47, no. 7, 1996, pages 363-366, XP000972181
- SCHWARTZ: "Modafinil for the treatment of excessive daytime sleepiness associated with narcolepsy" TODAY'S THERAPEUTIC TRENDS, vol. 16, no. 4, 1998, pages 287-308, XP000972129
- BEUSTERIEN: "Health-related quality of life effects of modafinil for treatment of narcolepsy" SLEEP, vol. 22, no. 6, 15 septembre 1999 (1999-09-15), pages 757-765, XP000972002
- BILLIARD: "Modafinil : a double-blind multicentric study" SLEEP, vol. 17, no. SUPPL.8, 1994, pages S107-S112, XP000972179
- BILLIARD: "Narcolepsie" REVUE DU PRATICIEN, vol. 46, no. 20, 15 décembre 1996 (1996-12-15), pages 2428-2434, XP000972127
- ARNULF: "Modafinil in obstructive sleep apnea-hypopnea syndrome : a pilot study in 6 patients" RESPIRATION, vol. 64, no. 2, 1997, pages 159-161, XP000972006 Paris, FRANCE
- TERZOUDI, MARIA (1) ET AL: "Fatigue in multiple sclerosis: Evaluation and a new pharmacological approach." NEUROLOGY, ( APRIL 11, 2000 ) VOL. 54, NO. 7 SUPP. 3, PP. A61-A62. MEETING INFO.: 52ND ANNUAL MEETING OF THE AMERICAN ACADEMY OF NEUROLOGY. SAN DIEGO, CA, USA APRIL 29-MAY 06, 2000 AMERICAN ACADEMY OF NEUROLOGY. , XP001036618
- DATABASE DRUGNL [en ligne] STN AN=2000:549, XP002185881 & R&D FOCUS DRUG NEWS, 14 février 2000 (2000-02-14),
- HOHLFELD R: "["Fatigue" in multiple sclerosis]. "Fatigue" bei multipler Sklerose. Erganzende Bemerkung zum Beitrag Zimmermann u. Hohlfeld aus Nervenarzt (1999) 70: 566-574." NERVENARZT, (2000 AUG) 71 (8) 686. , XP001050528
- RAMMOHAN, KOTTIL W. (1) ET AL: "Modafinil: Efficacy and safety for the treatment of fatigue in patients with multiple sclerosis." NEUROLOGY, ( APRIL 11, 2000 ) VOL. 54, NO. 7 SUPP. 3, PP. A24. MEETING INFO.: 52ND ANNUAL MEETING OF THE AMERICAN ACADEMY OF NEUROLOGY. SAN DIEGO, CA, USA APRIL 29-MAY 06, 2000 AMERICAN ACADEMY OF NEUROLOGY. , XP001036620
- DAVIS W.M.: "Multiple sclerosis: Continuing mysteries and current management." DRUG TOPICS, ( 19 JUN 2000 ) 144/12 (93-102). , XP001036734
- FERRARO L ET AL: "THE VIGILANCE PROMOTING DRUG MODAFINIL INCREASES EXTRACELLULAR GLUTAMATE LEVELS IN THE MEDIAL PREOTIC AREA AND THE POSTERIOR HYPOTHALAMUS OF THE CONSCIOUS RAT: PREVENTION BY LOCAL GABA4 RECEPTOR BLOCKADE" NEUROPSYCHOPHARMACOLOGY, ELSEVIER SCIENCE PUBLISHING, NEW YORK, NY, US, vol. 20, no. 4, avril 1999 (1999-04), pages 346-356, XP000972145 ISSN: 0893-133X
- DATABASE MEDLINE [en ligne] US NATIONAL LIBRARY OF MEDICINE (NLM), BETHESDA, MD, US; STN AN= 97249489, AKERSTEDT T ET AL: "Alertness-enhancing drugs as a countermeasure to fatigue in irregular work hours." retrieved from STN Database accession no. 97249489 XP002185882 & CHRONOBIOLOGY INTERNATIONAL, (1997 MAR) 14 (2) 145-58. REF: 81 ,
- ANTONINI ET AL: 'Selegiline in the treatment of hypersomnolence in myotonic dystrophy: a pilot study' J. NEUROL. SCI. vol. 147, no. 2, 1997, pages 167 - 169

## Description

La présente invention concerne une nouvelle utilisation en thérapeutique du modafinil et de ses dérivés.

Le modafinil ou benzhydrylsulfinyl acétamide est un composé de formule :

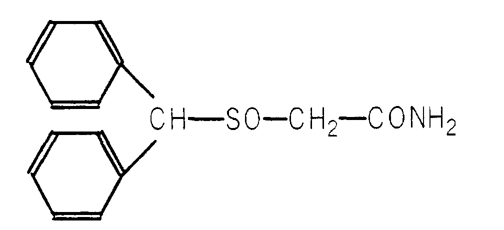

Ce composé et son application thérapeutique comme agent actif sur le système nerveux central ont été décrits dans la demande de brevet FR-A-2 385 693.

Le modafinil est utilisé en thérapeutique dans le traitement des narcolepsies et des hypersomnies idiopathiques.

Billiard, La revue du praticien, 46(20); 2428-2434, 1996 décrit l'utilisation du modafinil dans le traitement des narcolepsies.

La demande de brevet W 0 112 170 décrit l'utilisation du modafinil dans le traitement du THADA et de la fatigue associée à la sclérose en plaques.

Antonini et al, J. Neurol. Sci., 147(2); 167-169, 1997 décrit l'échec du traitement de l'hypersomnolence chez les patients souffrant d'une myotonie dystrophique par la sélégiline, qui est un médicament pourtant efficace dans le traitement de l'hypersomnolence de la narcolepsie.

Les isomères lévogyre et dextrogyre du modafinil ont été en outre décrits dans EP-A-0 233 106, l'isomère lévogyre étant présenté comme ayant une meilleure biodisponibilité.

On a maintenant découvert que le modafinil et ses isomères possèdent un effet bénéfique sur les troubles de la vigilance associés aux myopathies et en particulier à la dystrophie myotonique.

La dystrophie myotonique ou maladie de Steinert, la plus fréquente des myotonie de l'adulte, est une maladie héréditaire dont la prévalence estimée est de l'ordre de 5 à 8/100.000 (P.S. Harper, in "Myotonic dystrophy", 2^{nd} Ed., Saunders, Philadelphia, USA, 1989). Cette affection, transmise selon un mode autosomique dominant, est constamment associée à une anomalie génétique (localisée sur le chromosome 19) qui est caractérisée par une amplification anormale d'une séquence trinucléotidique appartenant à un gène codant pour une protéine kinase.

La dystrophie myotonique est une pathologie multisystémique pouvant comporter une atteinte des muscles squelettiques et respiratoires, du coeur, du système endocrinien, du cristallin et du système nerveux central. Elle est souvent révélée par l'atteinte musculo-squelettique qui comprend une myotonie spontanée ou provoquée, une atrophie et un déficit moteur touchant essentiellement les muscles du visage, les muscles mastiquateurs, les muscles sterno-cléido-mastoïdiens et la musculature distale des membres. L'atteinte neurologique (objectivée par IRM) comprend des lésions telles que dilatation ventriculaire, atrophie, modification de la substance blanche sous-corticale ainsi que des dégénérescences neurofibrillaires hippocampiques et des pertes neuronales. Cette atteinte neurologique se traduit, au plan clinique, par des troubles cognitifs pouvant aller jusqu'à un tableau de démence sous-corticale, un manque d'initiative et des troubles de la personnalité (indifférence, paresse, agressivité, ...)

L'atteinte de la fonction respiratoire et les troubles du sommeil sont habituels. Pour certains patients, la gêne principale exprimée est l'hypersomnolence diurne sévère (P.S. Harper, R. Rüdel, in "Myotonic Dystrophy", A. Engel and C. Franzini-Amstrong, eds. Myology 2^{nd} Edition, 1994). Les mécanismes de l'hypersomnie diurne de la dystrophie myotonique sont encore obscurs. Elle pourrait être en rapport avec une carbonarcose liée à une hypoventilation alvéolaire, à un syndrome d'apnées du sommeil centrales et/ou obstructives ou d'origine idiopathique. Cependant, elle peut être observée chez des patients dystrophiques ayant un bilan d'explorations fonctionnelles respiratoires normal et une polysomnographie également normale. Dans cette situation, la dystrophie myotonique de Steinert résulterait d'une atteinte du système nerveux central qui n'est pas évoquée dans les tableaux classiques de maladie de Gélineau (narcolepsie), d'apnées du sommeil ou d'hypersomnie idiopathique (Van der Meché et al., Neurol. Neurosurg Psychiatry, 57 : 626-628, 1994 ; Ono et al., Neurology , 46 : 228-231, 1986).

L'hypersomnolence diurne des patients atteints de la maladie de Steinert (dystrophie myotonique) a déjà fait l'objet de quelques tentatives thérapeutiques à l'aide de médicaments présentant des propriétés de stimulation du système nerveux central.

La littérature médicale disponible à ce jour présente des résultats préliminaires obtenus avec deux composés de mécanismes d'action et de profils pharmacologiques différents : le méthylphénidate (Van der Meché et al., Muscle and Nerve 9 : 341-344, 1986) et la sélégiline (Antonini et al., J. Neurol. Sciences 147 : 167-169, 1997). Dans le premier cas, une amélioration du niveau d'éveil a été constatée, tandis que l'utilisation de l'inhibiteur de la MAO-B s'est révélée inefficace à la dose de 20 mg/jour.

Ce contexte, peu favorable, de tentatives thérapeutiques est peut-être à rapprocher de la spécificité physio-pathologique de la dystrophie myotonique, surtout lorsque celle-ci est accompagnée des lésions du SNC qui ne sont retrouvées ni dans la maladie de Gélineau, ni dans les hypersomnies idiopathiques, ni dans les apnées du sommeil.

Le modafinil diffère de manière importante des psychostimulants conventionels (d-amphétamine, méthylphénidate) au plan de l'activité neurochimique. En effet, dans les gammes de doses qui modifient l'éveil, le modafinil n'agit pas, au contraire de la d-amphétamine et du méthylphénidate, sur la dopamine qui est la principale cible des psychostimulants conventionnels. Le modafinil diffère également de l'amphétamine en termes d'activité comportementale. Dans les tests de discrimination de substances addictives, la généralisation des effets du modafinil à l'amphétamine est très faible ; le modafinil ne provoque ni auto-administration ni préférence de place, deux comportements rapidement développés par les psychostimulants conventionnels. De plus, le modafinil, ne modifie pas l'auto-administration de cocaïne et n'influence pas la rechute de ce comportement. Enfin, si le modafinil peut activer l'expression du proto-oncogène c-fos dans le tissu cérébral, il le fait dans des zones tout à fait distinctes de celles concernées par les psychostimulants conventionnels. Ainsi, dans une publication de JS. Lin et al. (PNAS, 1996), le proto-oncogène c-fos n'est pas exprimé dans les zones de projections dopaminergiques centrales (dont le cortex), après administration de doses éveillantes de modafinil chez le chat, alors qu'il se trouve fortement exprimé dans ces régions après administration de doses équiéveillantes (à celles de modafinil) de d-amphétamine et de méthylphénidate.

Ayant ces schémas de pensée et ces données expérimentales en tête, les inventeurs ont découvert, de manière inattendue, que le modafinil administré à des malades atteints de dystrophie myotonique permettait de restaurer un niveau de vigilance diurne compatible avec une vie psychosociale relationnelle satisfaisante. Ces résultats positifs apparaissent d'autant plus inattendus que des patients souffrant de la maladie de Steinert ont des explorations fonctionnelles respiratoires normales et des bilans de polysomnographie normaux, situations qui militent en faveur d'un effet original du modafinil, indépendant de ce qui a déjà été découvert et qui a fait l'objet de brevets d'utilisation thérapeutiques déjà individualisés.

Les inventeurs ont découvert plus généralement que le modafinil et ses isomères permettaient de corriger les troubles de la vigilance qui sont observés au cours de l'évolution des myopathies.

La présente invention a donc pour objet I' utilisation du modafinil et de ses isomères pour la fabrication d'un médicament destiné à corriger les troubles de la vigilance observés au cours de l'évolution des myopathies.

La présente invention a plus particulièrement pour objet I' utilisation du modafinil et de ses isomères pour la fabrication d'un médicament destiné à corriger les troubles de la vigilance associés à la dystrophie myotonique ou maladie de Steinert.

Chez les patients atteints de myopathies et en particulier de dystrophie myotonique, le modafinil et ses isomères peuvent être utilisés pour lutter spécifiquement contre les hypersomnolences diurnes, pour restaurer une architecture de sommeil comparable à celle du sommeil normal et améliorer la qualité de vie psychosociale et relationnelle.

Le modafinil et ses isomères peuvent être administrés sous une forme convenant pour l'administration par voie orale et contenant le modafinil ou chacun de ses isomères à des doses unitaires comprises entre 50 et 600 mg, de préférence 100 à 400 mg.

Les formes pharmaceutiques préférées sont les formes pharmaceutiques destinées à une administration orale répétée sous forme de comprimés sécables ou non, de gélules, de capsules ou de microgranules à vitesse contrôlée.

Le modafinil peut toutefois être administré par d'autres voies, en particulier par voie transdermique, et être présenté sous forme d'onguents, pommades, gels, solutions, lotions, de formes liquides formant des pellicules sur la peau pour l'administration transdermique, ou de formes de type "patch".

Ainsi, dans une série de malades souffrant de dystrophie myotonique, chez lesquels les méthodes validées d'étude des perturbations du cycle veille/sommeil ont mis en évidence des hypersomnies diurnes de fréquence et durée considérées comme pathologiques (au regard de l'expérience clinique et thérapeutique des apnées du sommeil et de la narcolepsie), ont été traités par des doses orales de modafinil comprises entre 100 et 400 mg/jour. Chez la majorité de ces malades, le traitement par modafinil s'est révélé subjectivement efficace, les patients rapportent une nette régression des troubles de la vigilance et de la somnolence diurnes.

A titre d'exemple, chez un malade (âgé de 34 ans), atteint de maladie de Steinert, caractérisée entre autre, par une atrophie temporale, un ptosis, une myotonie associée à une faiblesse musculaire, des troubles du comportement, et une somnolence (dont le début remontait à 1988) avec paralysies du sommeil, sans hallucinations hypnagogiques ni attaques de cataplexie, il a été observé une diminution de 18 unités sur l'échelle de somnolence d'Epworth (voir Johns M.W. Sleep, 1991, 14, 540) après traitement à la dose orale de 100mg par jour de modafinil pendant six mois, entre février 1999 (score sur l'échelle d'Epworth = 20) et Août 1999 (score sur l'échelle d'Epworth = 2).

Le bénéfice obtenu par un traitement au modafinil a également été démontré chez deux autres patients souffrant de maladie de Steinert. Le premier patient est une femme de 39 ans, présentant un morphotype évocateur avec atrophie des fosses temporales, ainsi qu'un déficit des membres inférieurs, une faiblesse cervicale et une myotrophie diffuse. Atteinte d'hypersomnie diurne sévère se traduisant par un score de 16 sur l'échelle d'Epworth et de 8 sur l'échelle du Karolinska ou échelle KSS (Akerstedt P. Gillberg M. , Int. J. Neurosci. 1990 ; 52 : 29-37), cette patiente présentait - après 2 ans de traitement par modafinil (200 mg par jour) - une disparition complète ou presque complète de la symptomatologie (score sur l'échelle de somnolence KSS : avant : 8/après : 2) et une nette amélioration de sa qualité de vie personnelle et professionnelle. Egalement atteint des symptômes de la maladie de Steinert avec une myotonie des deux mains, un déficit musculaire et une cataracte, le second patient (âgé de 38 ans) présentait sans traitement une hypersomnie diurne très prononcée (Echelle d'Epworth = 16). L'administration de modafinil (600 mg/jour) a induit une résolution partielle de la somnolence diurne (score sur l'échelle de somnolence KSS : avant : 8/après : 4).

Traité par le modafinil (200 mg/jour), un quatrième patient (âgé de 25 ans) au morphotype évocateur de la maladie de Steinert avec faciès allongé, atrophie des fosses temporales, prognathisme, macroglossie et dysarthrie et présentant, également des signes intenses de myotonie diffuse, a vu son hypersomnolence diurne nettement améliorée (échelle d'Epworth : avant: 11/après : 6 ; échelle Karolinska : avant : 7/après : 2) au bout de 2 mois d'administration journalière de modafinil.

Les symptômes d'hypersomnolence diurne intense observés chez un cinquième patient de 40 ans atteint de dystrophie mytonique avec signes myogènes et d'une polyneuropathie surajoutée ont régressé sous traitement par le modafiinil (300 mg/j), ce bénéfice se traduisant par une nette amélioration de son score sur l'échelle d'Epworth (avant : 19/après : 10).

Une autre amélioration clinique a également été observée sous modafinil, chez un sixième patient qui consulte en 1990 pour somnolence diurne excessive évoluant depuis un an. L'examen clinique de ce patient révèle des signes de la maladie de Steinert. Traité sans succès par le méthylphénidate (dérivé d'amphétamine), ce patient de 56 ans a reçu, à la place, 200 mg/jour puis 300 mg/jour de modafinil en prise orale. Au bout d'un 1 mois et demi de ce nouveau traitement, l'hypersomnolence de ce sixième patient a été nettement améliorée (score sur l'échelle de somnolence d'Epworth (avant : 18-24/après : 15).

## Revendications

1. Utilisation du modafinil et de ses isomères pour la fabrication d'un médicament destiné à corriger les troubles de la vigilance observés au cours de l'évolution des myopathies.

2. Utilisation du modafinil et de ses isomères pour la fabrication d'un médicament destiné à corriger les troubles de la vigilance associés à la dystrophie myotonique ou maladie de Steinert.

3. Utilisation du modafinil et de ses isomères pour la fabrication d'un médicament pour lutter spécifiquement contre les hypersomnolences diurnes chez des patients souffrant de myopathies et en particulier de dystrophie myotonique.

4. Utilisation du modafinil et de ses isomères pour la fabrication d'un médicament destiné à restaurer une architecture de sommeil comparable à celle du sommeil normal et améliorer la qualité de vie psychosociale et relationnelle chez des patients souffrant de myopathies et en particulier de dystrophie myotonique.

5. Utilisation selon l'une quelconque des revendications 1 à 4, **caractérisée en ce que** lesdits médicaments sont sous une forme convenant pour l'administration par voie orale et contiennent le modafinil à des doses unitaires comprises entre 50 et 600 mg, de préférence 100 à 400 mg.

6. Utilisation selon la revendication 5, **caractérisée en ce que** les formes pharmaceutiques utilisées sont destinées à une administration orale répétée sous forme de comprimés sécables ou non, de gélules, de capsules ou de microgranules à vitesse contrôlée.

7. Utilisation selon l'une quelconque des revendications 1 à 4 **caractérisée en ce que** lesdits médicaments sont sous une forme convenant pour l'administration transdermique.

## Patentansprüche

1. Verwendung von Modafinil und seiner Isomeren zur Herstellung eines Arzneimittels, das zur Korrektur von Störungen der Wachheit bestimmt ist, die im Laufe der Entwicklung von Myopathien beobachtet werden.

2. Verwendung von Modafinil und seiner Isomeren zur Herstellung eines Arzneimittels, das zur Korrektur von Störungen der Wachheit bestimmt ist, die mit der myotonen Dystrophie oder der Steinert-Krankheit verbunden sind.

3. Verwendung von Modafinil und seiner Isomeren zur Herstellung eines Arzneimittels zur spezifischen Bekämpfung von Hypersomnolenzen am Tage bei Patienten, die unter Myopathien und insbesondere an der myotonen Dystrophie leiden.

4. Verwendung von Modafinil und seiner Isomeren zur Herstellung eines Arzneimittels, das zur Wiederherstellung der Schlafarchitektur, vergleichbar dem Normalschlaf, und zur Verbesserung der psychosozialen und Beziehungen betreffenden Lebensqualität bei Patienten bestimmt ist, die an Myopathien und insbesondere an der myotonen Dystrophie leiden.

5. Verwendung nach einem der Ansprüche 1 bis 4, **dadurch gekennzeichnet, daß** diese Medikamente in einer zur oralen Verabreichung üblichen Form vorliegen und Modafinil in Einzeldosen zwischen 50 und 600 mg, vorzugsweise 100 bis 400 mg, enthalten.

6. Verwendung nach Anspruch 5, **dadurch gekennzeichnet, daß** die verwendeten pharmazeutischen Zubereitungen zur wiederholten Verabreichung mit einer kontrollierten Geschwindigkeit in Form von teilbaren oder nicht teilbaren Komprimaten, Pillen, Kapseln oder Mikrogranulaten bestimmt sind.

7. Verwendung nach einem der Ansprüche 1 bis 4, **dadurch gekennzeichnet, daß** diese Medikamente in einer für die transdermale Verabreichung üblichen Form vorliegen.

## Claims

1. Use of modafinil and the isomers thereof for producing a drug intended to correct the problems of wakefulness observed during the development of myopathies.

2. Use of modafinil and the isomers thereof for producing a drug intended to correct the problems of wakefulness associated with myotonic dystrophy or Steinert's disease.

3. Use of modafinil and the isomers thereof for producing a drug for specifically combating diurnal hypersomnolence in patients suffering from myopathies and particularly myotonic dystrophy.

4. Use of modafinil and the isomers thereof for producing a drug intended to restore a sleep structure comparable with that of normal sleep and improve the psychosocial and relationship quality of life in patients suffering from myopathies and particularly myotonic dystrophy.

5. Use according to any one of claims 1 to 4, **characterised in that** the drugs are in a form suitable for oral administration and contain modafinil in single doses of between 50 and 600 mg, preferably 100 to 400 mg.

6. Use according to claim 5, **characterised in that** the pharmaceutical forms used are intended for repeated oral administration in the form of tablets which may or may not be divisible, gelatine or other capsules or microgranules with sustained release.

7. Use according to any one of claims 1 to 4, **characterised in that** the drugs are in a form suitable for transdermal administration.
